(19) Europäisches Patentamt

European Patent Office

Office européen des brevets

(11) Veröffentlichungsnummer: **0 402 638 B1**

(12) **EUROPÄISCHE PATENTSCHRIFT**

(45) Veröffentlichungstag der Patentschrift: **02.03.94**

(21) Anmeldenummer: **90109080.3**

(22) Anmeldetag: **14.05.90**

(51) Int. Cl.⁵: **G01N 1/12**, G01N 25/02, G01K 13/12

(54) **Vorrichtung zur Probenentnahme und zur Bestimmung der Liquiduskurve einer Metallschmelze.**

(30) Priorität: **14.06.89 DE 3919362**

(43) Veröffentlichungstag der Anmeldung:
**19.12.90 Patentblatt 90/51**

(45) Bekanntmachung des Hinweises auf die
Patenterteilung:
**02.03.94 Patentblatt 94/09**

(84) Benannte Vertragsstaaten:
**AT DE ES FR GB IT NL SE**

(56) Entgegenhaltungen:
**AT-B- 319 639**
**US-A- 3 656 346**
**US-A- 3 813 944**
**US-A- 4 557 152**

(73) Patentinhaber: **Heraeus Electro-Nite International N.V.**
**Amerikalei 110, bus 5**
**B-2000 Antwerpen(BE)**

(72) Erfinder: **Baerts, Christiaan Eugene Edouad**
**Meldertsesteenweg 142**
**B-3940 Beringen-Paal(BE)**

(74) Vertreter: **Kühn, Hans-Christian**
**Heraeus Holding GmbH,**
**Stabsstelle Schutzrechte,**
**Heraeusstrasse 12-14**
**D-63450 Hanau (DE)**

EP 0 402 638 B1

**Beschreibung**

Die Erfindung betrifft eine Vorrichtung zur Ermittlung des Kohlenstoffgehaltes von Stahl mit einem im Bereich von 0,04 bis 0,4 % liegenden Kohlenstoffgehalt durch Messung des Liquiduspunktes, wobei die maximale Differenz zwischen der Temperatur des flüssigen Metalls und dessen Erstarrungstemperatur 250° C beträgt, bestehend aus einer verbrauchbaren Meßsonde mit einer Erstarrungskammer, die mit einer Einlauföffnung für das flüssige Metall und einem Temperaturmeßfühler versehen ist, der an dem der Einlauföffnung entgegengesetzten Ende in die Kammer hineinragt und mit dem der Verlauf der Abkühlung des flüssigen Metalls über einen bestimmten Zeitraum ermittelt wird.

Vorrichtungen zur Bestimmung von Phasenübergängen sind beispielsweise aus der DE-AS 1 648 964 und der US-PS 4 557 152 bekannt. Sie werden insbesondere zur Bestimmung des Kohlenstoffgehaltes von in LD-Konvertern hergestelltem Stahl benutzt. Die Erstarrungskammern werden im allgemeinen aus Hohl- oder Formkörpern aus keramischem Material aus mit Harz überzogenem Sand, aus Metall oder einer Kombination dieser Stoffe hergestellt. Die Erfindung geht als Stand der Technik von der aus der US-PS 4 557 152 bekannten Sonde aus, bei der die Erstarrungskammer in einem aus feuerfestem Formsand bestehenden Hohlkörper ausgebildet ist, in den ein oder zwei Metallplatten als Kühlelemente eingelegt sind, die einen Teil der Seitenwandung der Kammer bilden, während der nicht mit Metallplatten belegte Teil der Seitenwandung sowie die vordere und hintere Wandung der Kammer von dem feuerfesten Formsand begrenzt werden.

Aus der AT 319639 ist eine Vorrichtung zum Schnellbestimmen von Sauerstoff und/oder Kohlenstoff in Metallschmelzen mit Hilfe der thermischen Analyse bekannt. Die Vorrichtung weist zwei Tauchkokillen mit je einem in einen Probenraum mündenden Einlauf auf. In jedem Probenraum ist ein Thermoelement angeordnet und in einem der Probenräume befindet sich als Desoxidationsmittel ein Aluminium-Draht.

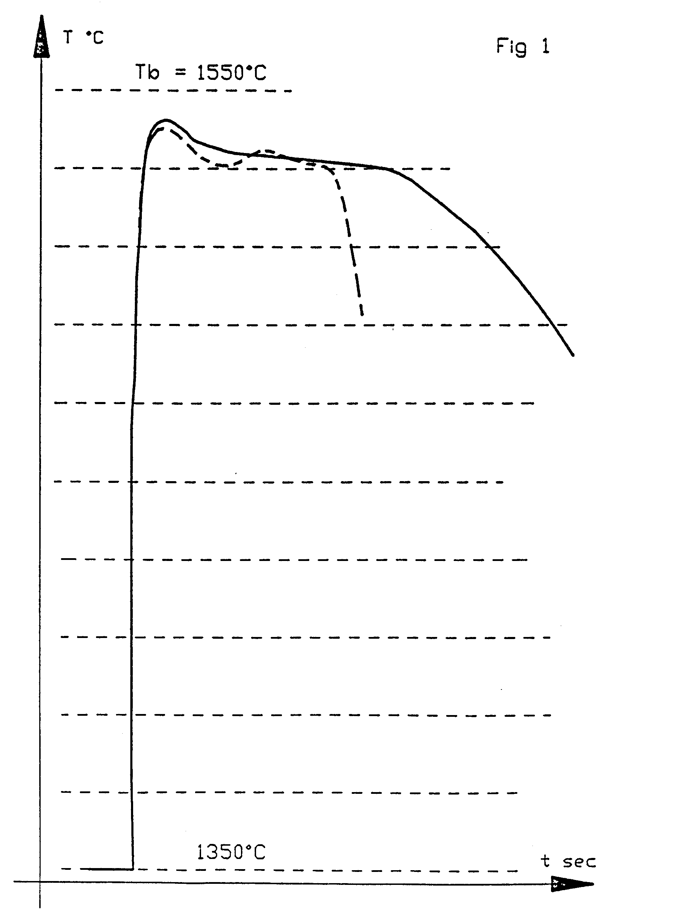

Beim praktischen Einsatz der Sonden hat sich herausgestellt, daß bei der Verwendung der bekannten Erstarrungskammern nur selten ein deutlich erkennbarer Haltepunkt ermittelt werden kann, wodurch die Genauigkeit der Kohlenstoffbestimmung beeinflußt wird. Dies ist unter anderem darauf zurückzuführen, daß einer Abweichung von 1° C eine Änderung des Kohlenstoffgehaltes von 0,013 % C entspricht. Für die Durchführung von Messungen in Stahl mit einem Kohlenstoffgehalt von 0,02 bis 0,2 % C führt dies zu beträchtlichen Fehlern. In Figur 1 ist mit gestrichelten Linien ein typischer Kurvenverlauf der Abkühlungskurve einer Stahlprobe dargestellt, der bei Verwendung einer aus keramischem Material, d. h. einem Material mit schlechter Wärmeleitfähigkeit bestehenden Erstarrungskammer erzielt wird. Die Erstarrungstemperatur wird durch die schlechte Wärmeabfuhr nur langsam erreicht, so daß der Liquidus-Haltepunkt nur schwierig festgelegt werden kann und die Bestimmung von Phasenübergängen im Festzustand praktisch unmöglich ist. Mit punktierten Linien ist in Figur 1 der Kurvenverlauf aufgezeichnet, der mit einer Erstarrungskammer ermittelt wurde, die aus Metall, d. h. einem Material mit guter Wärmeleitfähigkeit besteht. Die Kurven zeigen häufig typisches Unterkühlungs-Phänomen, das sich mit einem S-förmigen Verlauf im Bereich der Erstarrungstemperatur äußert und die Bestimmung des Liquidus-Haltepunktes erheblich erschwert.

Aus der US 3,813,944 ist ein Probennehmer für Metallschmelzen bekannt mit einem Probenraum, in dem ein Thermoelement angeordnet ist. Der Probennehmer dient der thermischen Analyse von nodulairem Gußeisen bei Temperaturen im Bereich von etwa 1010 C° bis 1260° C.

Die Aufgabe, die der Erfindung zugrunde liegt, besteht darin, die Erstarrungskammer eines Probennehmers so auszubilden, daß der Erstarrungsprozeß homogener verläuft, d. h. die Temperaturgradienten innerhalb der Probe während des Erstarrens so klein wie möglich gehalten werden, um Abkühlungskurven zu enthalten, die eine genauere Bestimmung des Kohlenstoffgehaltes mit hoher Stetigkeit und Gleichmäßigkeit sicherstellen.

Der Erfindung liegt die Erkenntnis zugrunde, daß die Genauigkeit der Messungen erheblich gesteigert werden kann, wenn der die Erstarrungskammer bildende Hohlkörper bestimmte Abmessungen aufweist und seine Wandung und die diese umgebende Umhüllung aus nach besonderen Kriterien ausgewählten Materialien bestehen.

Ausgehend von einer Vorrichtung der eingangs beschriebenen Art besteht die Erfindung in der gleichzeitigen Anwendung der folgenden Merkmale:

a) Die Seitenwandung der Erstarrungskammer ist aus einem die Kammer vollständig umschließenden, sich in Längsrichtung der Meßsonde erstreckenden Metallmantel aus Stahl mit einem Kohlenstoffgehalt von etwa 0,04 %, gebildet,

b) die beiden offenen Enden des Mantels sind mit einem Material abgedeckt, dessen Wärmeleitfähigkeit wesentlich niedriger ist als die des Mantels,

c) der Metallmantel ist auf der der Erstarrungskammer abgewandten Seite vollständig von einem Material umschlossen, dessen Wärmeleitfähigkeit wesentlich niedriger ist als die des Metalls des Mantels,

d) die Masse des Metallmantels ist so bemessen, daß das Verhältnis der Masse der Stahlprobe ($M_p$) zur Masse des Metallmantels ($M_m$) im Bereich von 1,8 bis 2,6 liegt, wobei die Wärme, die von der in die Erstarrungskammer einfließenden Schmelze abgegeben und von dem Metallmantel aufgenommen wird, im Bereich des Temperaturmeßfühlers zu einer Gleichgewichtstemperatur führt, die der Erstarrungstemperatur soweit wie möglich angenähert ist.

Zeit-Temperaturdiagramme können als Idealkurven aufgezeichnet werden, wenn die Parameter vorher festgelegt werden, d. h. wenn die Badtemperatur und die Erstarrungstemperatur bekannt sind. Dies ist in der Praxis nicht der Fall. Für die erfindungsgemäße Ausbildung der Erstarrungskammer kommt es darauf an, daß die Masse des Metallmantels in einem bestimmten Verhältnis zur Masse der einlaufenden Schmelze steht, um sicherzustellen, daß in dem Mantel soviel Wärmeenergie gespeichert wird, daß die Temperatur der metallischen Wandung beim Erreichen des Phasenüberganges so nahe wie möglich bei der Phasenübergangstemperatur liegt. Die in dem Metallmantel der Erstarrungskammer gespeicherte Wärmeenergie ist außer von dem Volumen der Probekammer von der Differenz zwischen der Badtemperatur und der Erstarrungstemperatur abhängig. Optimale Abmessungen, d. h. ein optimales Verhältnis zwischen der Masse des Metallmantels und der Masse der Probe lassen sich daher nur für bestimmte Temperaturbereiche und Kohlenstoffgehalte errechnen. In der Praxis variiert die Differenz zwischen Bad- und Erstarrungskammer zwischen maximal 50 bis 250° C, was einer Schwankung der Badtemperatur von 1570 bis 1780° C bzw. einem Kohlenstoffgehalt von 0,6 bis 0,02 % C entspricht. Dabei tritt die angegebene obere Temperaturgrenze nur in seltenen Fällen auf, die am häufigsten vorkommende maximale Temperatur liegt bei etwa 1700° C.

Mit der folgenden Formel (I), die das thermodynamische Gleichgewicht zwischen der Masse der Metallwandung und der Masse der in der Erstarrungskammer befindlichen Probe wiedergibt, kann die für die erfindungsgemäße Ausbildung des Mantels erforderliche Masse gemäß Merkmal d) und damit das Verhältnis der Masse der Probe zur Masse des Mantels errechnet und bestimmt werden.

$$M_W \cdot \left[ \sum_{T=T_o}^{\substack{T_{yW} \\ T_e}} \int C_{pW} \cdot dT + \sum_{T\ T_o}^{T_e} L_{tW} \right] = M_p \left[ \sum_{T=T_e}^{\substack{T_{yP} \\ T_b \\ T_{xP}}} \int C_{pP} \cdot dT + \sum_{T=T_e}^{T_b} L_{tP} \right]$$

$$(I)$$

$M_w$ =       Masse der Metallwandung

$M_p$ =       Masse der Probe

$T_e$ =       Gleichgewichtstemperatur

$T_o$ =       Umgebungstemperatur

$T_b$ =       Badtemperatur

$C_p w$ =       Spezifische Wärmekapazität der Metallwandung

$C_p p$ =       Spezifische Wärmekapazität der Probe

$L_t w$ =       Latente Wärme der Metallwandung

$L_t p$ =       Latente Wärme der Probe

$T_y w - T_x w$ =       Temperaturunterschied zwischen zwei Phasenübergängen der Metallwandung

$T_y p - T_x p$ =       Temperaturunterschied zwischen zwei Phasenübergängen der Probe.

Eine bevorzugte Ausführungsform der Erfindung sieht vor, daß der Einlauf für die Schmelze aus einem Quarzrohr besteht, das nach außen zu mit einer Schlackenkappe abgedeckt ist und ganz oder teilweise von einem Metallrohr umgeben ist, das dicht in die Seitenwandung der Erstarrungskammer eingesetzt ist, wobei das Quarzrohr in dem Metallrohr mit feuerfestem Zement befestigt und im Inneren des Quarzrohres, falls erforderlich, ein Oxidationsmittel angeordnet ist.

Ferner können erfindungsgemäß die beiden offenen Enden des Mantels mit Stopfen verschlossen sein, wobei der obere Stopfen aus einem porösen Material besteht, das beim Einfüllen der Schmelze den Austritt von Luft aus der Probenkammer zuläßt. Erfindungsgemäß ist der untere, dem Eintauchende der Sonde zugewandte Stopfen, an dem der Temperaturmeßfühler angebracht ist, auf der der Erstarrungskammer zugewandten Seite wenigstens mit einer Schicht aus isolierendem feuerfesten Papier abgedeckt. Erfindungsgemäß ist der Abstand zwischen dem Mittelpunkt der Einlauföffnung und der Meßstelle des Meßfühlers mindestens halb so groß wie der Innendurchmesser des Mantels.

Vorzugsweise wird der Temperaturmeßfühler so angeordnet, daß er sich in einer mittleren Längsebene der Erstarrungskammer erstreckt. Der die Kammer umschließende Metallmantel besteht erfindungsgemäß aus einem Rohr mit vorzugsweise kreisförmigem Querschnitt.

Ausführungsbeispiel

Die Ausbildung einer erfindungsgemäßen Erstarrungskammer zur Bestimmung des Kohlenstoffgehaltes in einem Bereich von 0,04 bis 0,4 % C bei Verwendung eines Mantels aus Stahl mit einem Kohlenstoffgehalt von 0,04 % C erfolgt unter Berücksichtigung der folgenden Kriterien:

Bei einem Kohlenstoffgehalt des Metallbades von 0,04 % C ergibt die Berechnung unter Zugrundelegung der obigen Formel (I) ein optimales Massenverhältnis

$$Mv = \frac{Mp}{Mw} = 2,7$$

bei einer durchschnittlichen Badtemperatur von ca. 1700° C und einer vorausgesetzten Gleichgewichtstemperatur von 1528° C. Da die Gleichgewichtstemperatur von 1528° C oberhalb der Linie C-D des Eisen-Kohlenstoff-Diagrammes liegt, die den Übergang der δ-PHase in die Phase δ + flüssig begrenzt, würde der Mantel ganz oder teilweise zerstört werden. Man wählt daher eine Gleichgewichtstemperatur, die vorzugsweise etwa 2° C unterhalb der C-D-Linie liegt, was bei einem Kohlenstoffgehalt des Mantels von 0,04 % C einer Temperatur von 1513° C entspricht. Daraus läßt sich der Wärmeaustausch zwischen Probe und Wand berechnen und es ergeben sich die folgenden Werte:
Von der Schmelze abgegebene Wärme 22,3,
von der Wandung aufgenommene Wärme 58,4.
Daraus ergibt sich ein Massenverhältnis von

$$Mv = \frac{Mp}{Mw} = 2,6.$$

Bei einem Kohlenstoffgehalt des Metallbades von 0,4 % ergibt die Berechnung des Massenverhältnisses unter Zugrundelegung einer durchschnittlichen Badtemperatur von etwa 1600° C und einer Gleichgewichtstemperatur von 1504° C (2° unter der Erstarrungstemperatur) folgende Werte:
Von der Probe abgegebene Wärme 19,2,
von der Wandung aufgenommene Wärme 57,3.
Dies entspricht einem Massenverhältnis von:

$$Mv = \frac{Mp}{Mw} = 2,9.$$

Obschon das optimale Massenverhältnis bei einer Badtemperatur von 1600° C 2,9 beträgt, darf der Wert von 2,6 aus den vorstehend erwähnten Gründen nicht überschritten werden. Dieser Wert stellt somit die obere Grenze für den Fall dar, daß die Wandung der Probekammer aus Stahl mit einem Kohlenstoffgehalt von 0,04 % C besteht.

Bei praktischen Versuchen hat sich gezeigt, daß gute Resultate für den Kohlenstoffbereich von 0,04 bis 0,4 % C des Metallbads noch dann erzielt werden, wenn das Massenverhältnis $M_v = 1,8$ beträgt. Erfindungsgemäß wird daher für den vorstehend erwähnten Anwendungsfall ein Massenverhältnis gewählt, das zwischen 1,8 und 2,6 liegt.

Selbstverständlich können für den Mantel auch andere Materialien mit höherem Schmelzpunkt, wie z.B. Molybdän verwendet werden, so daß sich unter Zugrundelegung der obigen Berechnung ein anderes optimales Massenverhältnis ergeben kann.

Die Erfindung wird anhand der Zeichnung erläutert, auf der weitere Ausführungsformen der Erfindung dargestellt sind. Es zeigen:

Fig. 1       Abkühlungskurven (Zeit-Temperatur-Diagramme), die unter Verwendung von in bekannter Weise ausgebildeten Erstarrungskammern erhalten wurden,

Fig. 2a       einen Längsschnitt durch eine Meßsonde im Bereich einer Erstarrungskammer gemäß einer bevorzugten Ausführungsform der Erfindung,

Fig. 2b       einen Schnitt nach der Linie A-A der Fig. 2a,

Fig. 3          eine Abkühlungskurve (Zeit-Temperatur-Diagramm), die mit einer gemäß Fig. 2 ausgebildeten Erstarrungskammer erhalten wurde,

Fig. 4a        den Verlauf einer bei in der Praxis durchgeführten Messungen erhaltenen Abkühlungs- oder Erstarrungskurve im Bereich der Erstarrungstemperatur in in vergrößertem Maßstab unter Verwendung einer erfindungsgemäßen Erstarrungskammer,

Fig. 4b        eine entsprechende Kurve unter Verwendung einer Erstarrungskammer gemäß der US-PS 4 557 152,

Fig. 5a bis 5f     weitere Ausführungsformen für die erfindungsgemäße Ausbildung der Erstarrungskammer.

Fig. 1 zeigt zwei typische Abkühlungskurven, die unter Verwendung von bekannten Erstarrungskammern erhalten und einleitend erläutert wurden. Auf der Ordinate ist die Temperatur in Grad Celsius, auf der Abszisse die Zeit (t) in Sekunden aufgetragen. Mit (Tb) ist die Badtemperatur bezeichnet.

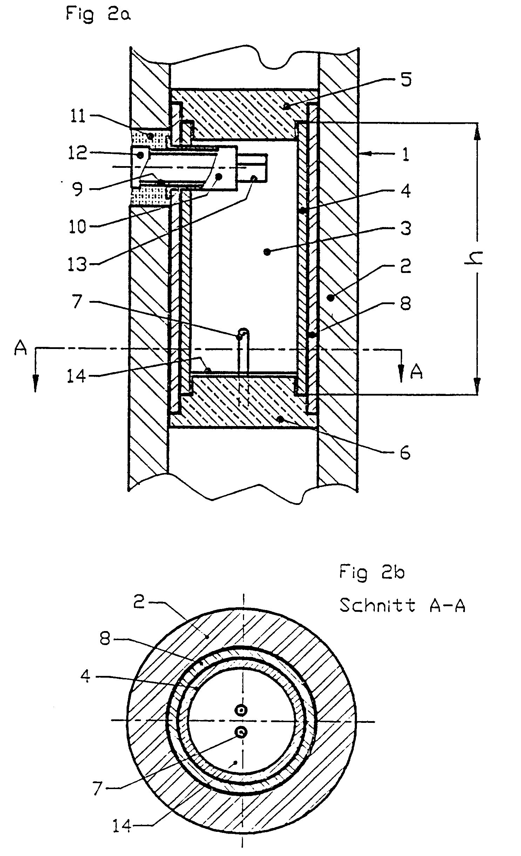

Die auf den Fig. 2a und 2b dargestellte Erstarrungskammer ist in einer Meßsonde (1) angeordnet, in deren vorderem (auf der Zeichnung unterem) nicht dargestellten Eintauchende weitere Meßeinrichtungen, wie z.B. zur Messung der Badtemperatur und zur Bestimmung des Sauerstoffgehaltes angeordnet sein können. Die Meßsonde (1) besteht aus einem äußeren Rohr (2) aus Pappe, in dem sich im Abstand vom Eintauchende die Erstarrungskammer befindet. Der Innenraum (3) der Kammer wird von einer Seitenwandung begrenzt, die aus einem sich in Längsrichtung der Meßsonde erstreckenden Mantel (4) mit der Höhe (h) und der Wandstärke (b) besteht, der aus Metall, d.h. einem Material mit hoher Wärmeleitfähigkeit gebildet wird und die Kammer vollständig umschließt. Bei dem dargestellten Ausführungsbeispiel besteht der Mantel aus einem Rohr mit kreisförmigem Querschnitt, dessen oberes und unteres Ende offen sind. Der Mantel kann auch einen anderen, z.B. quadratischen oder rechteckigen Querschnitt haben. Die beiden offenen Enden des Rohres sind mit Stopfen (5 u. 6) verschlossen. Der obere Stopfen (5) besteht aus einem porösen feuerfesten Material, das beim Einfüllen der Schmelze den Austritt von Luft und Gasen aus der Erstarrungskammer zuläßt.

Der untere Stopfen (6), der vorzugsweise aus keramischem Material besteht, enthält eine mittlere Bohrung, in die dicht ein Temperaturmeßfühler (7) eingesetzt ist, der vorzugsweise aus einem mit einem Thermoelement versehenen U-förmigen Quarzrohr bekannter Bauart besteht. Der Mantel (4) der Erstarrungskammer ist vollständig von einer weiteren Umhüllung (8) umschlossen, die vorzugsweise auch die Stopfen (5 u. 6) ganz oder teilweise umschließt und aus einem Material besteht, dessen Wärmeleitfähigkeit wesentlich niedriger ist als die des Metalls, aus dem der Mantel gebildet ist. Die Einlaßöffnung befindet sich an dem dem Eintauchende der Sonde abgewandten, auf der Zeichnung oberen Ende der Erstarrungskammer (3). Sie besteht aus einem Quarzrohr (9), das innerhalb eines Metallrohres (10) angeordnet ist, das von außen durch die Umhüllung (8) geführt und dicht in eine Öffnung der Seitenwandung der Erstarrungskammer eingesetzt und daran befestigt ist. Die Fixierung des Quarzrohres in dem Metallrohr erfolgt mit feuerfestem Zement (11) im Bereich des äußeren Papprohres, d.h. an einer Stelle, wo der Zement sich nicht störend auf das einlaufende Metall und die Probe auswirkt. Dies ist erforderlich, weil feuerfester Zement Kristallwasser enthält, der, wenn er mit dem flüssigen Metall in Berührung kommt, Gasentwicklungen und damit Porositäten hervorruft und sogar die chemische Zusammensetzung der Probe beeinflussen kann. Mit (12) ist eine verbrauchbare Schlackenkappe bezeichnet, die die Einlauföffnung beim Eintauchen der Sonde zunächst abdeckt, mit (13) ein Oxidationsmittel, das, falls erforderlich, im Auslaufende des Quarzrohres angeordnet wird. Der Stopfen (6), in dem der Temperaturmeßfühler befestigt ist, ist mit wenigstens einer Schicht (14) aus einem isolierenden feuerfesten Material, z.B. Aluminiumsilikat-Papier beschichtet.

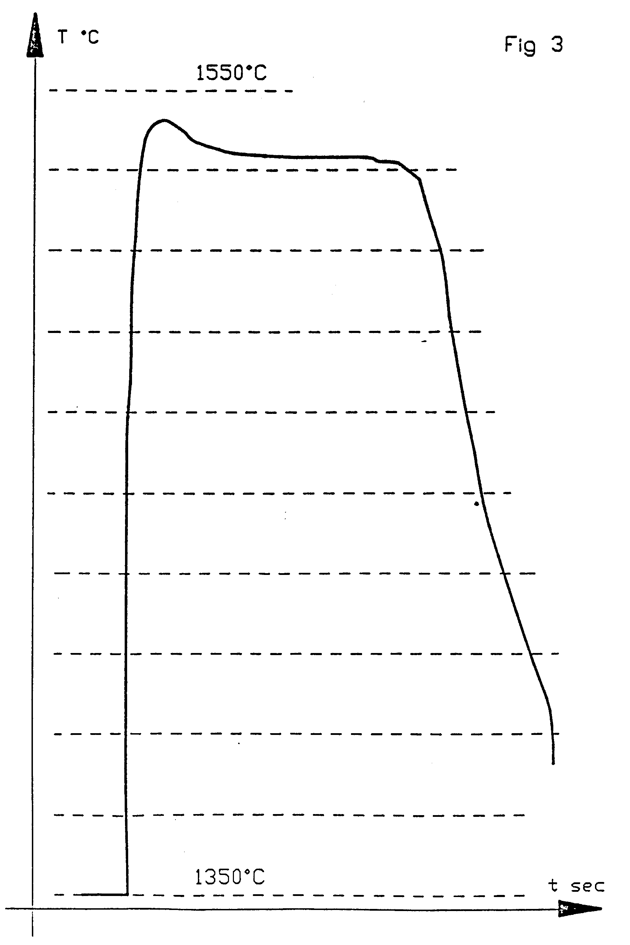

Fig. 3 zeigt eine Abkühlungskurve (Zeit-Temperatur-Diagramm), die unter Verwendung einer Erstarrungskammer erhalten wurde, wie sie in Zusammenhang mit Fig. 2 erläutert wurde. Die Kurve zeigt einen homogenen Verlauf mit einem nahezu waagerechten Abschnitt im Bereich der Erstarrungstemperatur, die sich mit großer Genauigkeit bestimmen läßt. Das Ergebnis wurde in zahlreichen Versuchen bestätigt. Mit der erfindungsgemäß ausgebildeten Erstarrungskammer läßt sich sowohl die Erstarrungstemperatur als auch die Phasenübergangstemperatur mit einem unerwartet hohen Grad an Genauigkeit und Reproduzierbarkeit bestimmen.

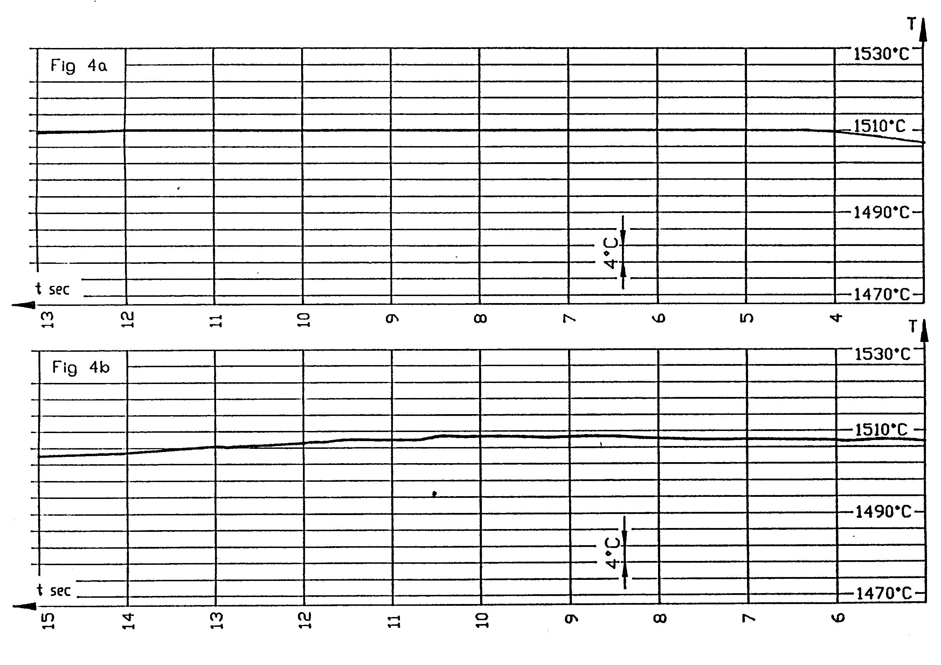

Die Fig. 4a und 4b zeigen Erstarrungskurven, die bei in der Praxis durchgeführten Messungen erhalten wurden, in vergrößertem Maßstab im Bereich der Erstarrungstemperatur. Die Kurve gemäß Fig. 4a wurde bei Verwendung einer erfindungsgemäß ausgebildeten Erstarrungskammer aufgezeichnet. Im Vergleich dazu zeigt Fig. 4b eine Kurve, die bei Verwendung einer Erstarrungskammer erhalten wurde, wie sie in der US-PS 4 557 152 beschrieben ist.

Während die Erstarrungskurve gemäß Fig. 4a einen praktisch waagerechten Verlauf bei einer Temperatur von 1510° C über nahezu 10 Sekunden zeigt, ist eine Temperaturabweichung von 2 bis 3° C, der Fehlmessungen im Bereich von 0,02 bis 0,04 % C entsprechen würden, bei der Kurve 4b deutlich erkennbar.

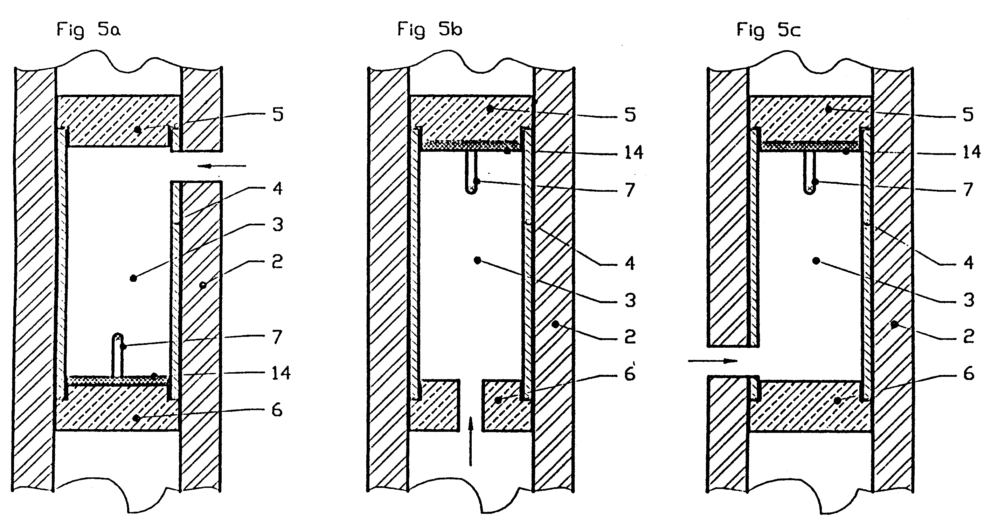

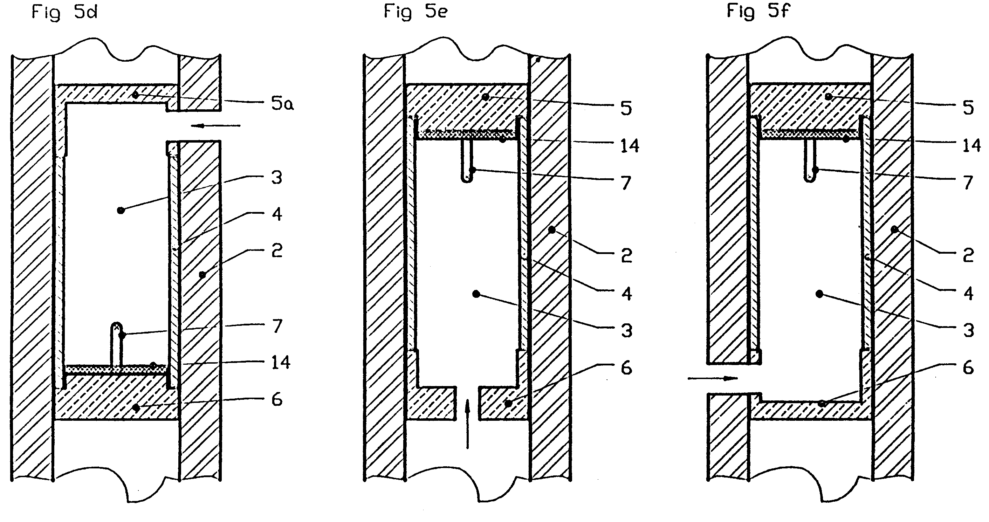

Auf den Fig. 5a bis 5f sind Längsschnitte durch sechs weitere Ausführungsformen für die Ausbildung von Erstarrungskammern schematisch dargestellt. Allen Ausführungsformen ist gemeinsam, daß das äußere Papprohr (2) direkt an der metallischen Seitenwandung (4) anliegt, die den Innenraum (3) der Erstarrungskammer seitlich begrenzt. Die übrige Ausbildung entspricht der der Fig. 2, wobei wie auch bei den übrigen Darstellungen der Fig. 5 für sich entsprechende Teile die gleichen Bezugszeichen benutzt und die Einlauföffnung schematisch dargestellt wurde. Bei den Ausführungsformen gemäß den Fig. 5b und 5c ist der Temperaturmeßfühler an dem oberen Stopfen (5) angeordnet, der auch mit der Beschichtung (14) abgedeckt ist. Die Einlauföffnung für die Schmelze befindet sich im unteren Bereich der Erstarrungskammer entweder in Form einer in dem Stopfen (6) angebrachten Öffnung (Fig. 5b) oder als Öffnung in der Seitenwandung im unteren Bereich der Kammer.

Die auf Fig. 5d dargestellte Erstarrungskammer entspricht im Prinzip der Ausführungsform gemäß Fig. 5a mit dem Unterschied, daß anstelle des Stopfens (5) eine tassenförmige Abdeckung (5a) aus porösem Material verwendet wird, die auf dem oberen Ende der zylindrischen Wandung des Mantels (4) aufliegt. Die Fig. 5e und 5f entsprechen im Prinzip den Fig. 5b und 5c, wobei lediglich der untere Stopfen (6) durch tassenförmige Elemente (6a) ersetzt wurde, die sich an das untere Ende des die Seitenwandung der Erstarrungskammer bildenden Mantels (4) anschließen und entweder ihrem Boden (Fig. 5e) oder in der Seitenwandung (Fig. 5f) mit einer Einlauföffnung versehen sind.

**Patentansprüche**

1. Vorrichtung zur Ermittlung des Kohlenstoffgehaltes von Stahl mit einem im Bereich von 0,04 bis 0,4 % liegenden Kohlenstoffgehalt durch Messungen des Liquiduspunktes, wobei die maximale Differenz zwischen der Temperatur des flüssigen Metalls und dessen Erstarrungstemperatur 250°C beträgt, bestehend aus einer verbrauchbaren Meßsonde mit einer Erstarrungskammer, die mit einer Einlauföffnung für das Metall und einem Temperaturmeßfühler versehen ist, der an dem der Einlauföffnung entgegengesetzten Ende in die Kammer hineinragt und mit dem der Verlauf der Abkühlung des flüssigen Metalls über einen bestimmten Zeitraum ermittelt wird, gekennzeichnet durch die gleichzeitige Anwendung der folgenden Merkmale:

   a) die Seitenwandung der Erstarrungskammer ist aus einem die Kammer vollständig umschließenden, sich in Längsrichtung der Meßsonde (1) erstreckenden Metallmantel (4) aus Stahl mit einem Kohlenstoffgehalt von etwa 0,04 %, gebildet,

   b) die beiden offenen Enden des Mantels sind mit einem Material (5, 6) abgedeckt, dessen Wärmeleitfähigkeit wesentlich niedriger ist als die des Metalls des Mantels,

   c) der Metallmantel (4) ist auf der der Erstarrungskammer abgewandten Seite vollständig von einem Material (2, 8) umschlossen, dessen Wärmeleitfähigkeit wesentlich niedriger ist als die des Metalls des Mantels(4),

   d) die Masse des Metallmantels (4) ist so bemessen, daß das Verhältnis der Masse der Stahlprobe $(M_p)$ zur Masse des Metallmantels $(M_w)$ im Bereich von 1,8 bis 2,6 liegt, wobei die Wärme, die von der in die Erstarrungskammer (3) einfließenden Schmelze abgegeben und von dem Metallmantel aufgenommen wird, im Bereich des Temperaturmeßfühlers (7) zu einer Gleichgewichtstemperatur führt, die der Erstarrungstemperatur soweit wie möglich angenähert ist.

2. Vorrichtung nach Anspruch 1, dadurch gekennzeichnet, daß der Einlauf für die Schmelze aus einem Quarzrohr (9) besteht, das nach außen zu mit einer Schlackenkappe (12) abgedeckt und ganz oder teilweise von einem Metallrohr (10) umgeben ist, das dicht in die Seitenwandung (4) der Erstarrungskammer (3) eingesetzt ist, wobei das Quarzrohr in dem Metallrohr mit feuerfestem Zement (11) befestigt und im Inneren des Quarzrohres, falls erforderlich, ein Oxidationsmittel (13) angeordnet ist.

3. Vorrichtung nach den Ansprüchen 1 und/oder 2, dadurch gekennzeichnet, daß die beiden offenen Enden des Metallmantels mit Stopfen (5, 6) verschlossen sind, wobei der obere Stopfen (5) aus einem porösen Material besteht , das beim Einfüllen der Schmelze den Austritt von Luft aus der Probekammer zuläßt.

4. Vorrichtung nach einem oder mehreren der vorhergehenden Ansprüche, dadurch gekennzeichnet, daß der untere, dem Eintauchende der Sonde zugewandte Stopfen (6), an dem der Temperaturmeßfühler (7) angebracht ist, auf der der Erstarrungskammer (3) zugewandten Seite mit wenigstens einer Schicht aus isolierendem feuerfesten Papier (14) abgedeckt ist.

**5.** Vorrichtung nach einem oder mehreren der vorhergehenden Ansprüche, dadurch gekennzeichnet, daß der Abstand zwischen dem Mittelpunkt der Einlauföffnung (9, 10) und der Meßstelle des Meßfühlers (7) mindestens halb so groß ist wie der Innendurchmesser des Metallmantels.

**6.** Vorrichtung nach einem oder mehreren der vorhergehenden Ansprüche, dadurch gekennzeichnet, daß der Temperaturmeßfühler (7) sich in einer mittleren Längsebene der Erstarrungskammer erstreckt.

**7.** Vorrichtung nach einem oder mehreren der vorhergehenden Ansprüche, dadurch gekennzeichnet, daß der die Kammer umschließende Metallmantel (4) aus einem Rohr mit vorzugsweise kreisförmigem Querschnitt besteht.

## Claims

**1.** A device to determine the carbon content of steel with a carbon content lying in the range of 0.04 to 0.4%, through measurements of the liquid point, in which the maximum difference between the temperature of the molten metal and its solidification temperature amounts to 250 °C, consisting of a consumable measurement probe with a solidification chamber, which is provided with an inlet opening for the metal and a temperature measurement sensor, which projects into the chamber at the end opposite the inlet opening and with which the progress of the cooling of the molten metal is determined over a particular period of time, characterised by the simultaneous application of the following features:
a) the side wall of the solidification chamber is formed from a metal casing (4) of steel with a carbon content of approximately 0.04%, completely surrounding the chamber and extending in longitudinal direction of the measurement probe (1),
b) the two open ends of the casing are covered with a material (5, 6), the heat conductivity of which is substantially lower than that of the metal of the casing,
c) the metal casing (4) is completely surrounded on the side facing away from the solidification chamber by a material (2, 8), the heat conductivity of which is substantially lower than that of the metal of the casing (4),
d) the mass of the metal casing (4) is proportioned such that the ratio of the mass of the steel sample ($M_p$) to the mass of the metal casing ($M_w$) lies in the range from 1.8 to 2.6, in which the heat which is given off from the melt flowing into the solidification chamber (3) and is received by the metal casing, leads in the region of the temperature measurement sensor (7) to an equilibrium temperature, which is as close as possible to the solidification temperature.

**2.** A device according to Claim 1, characterised in that the inlet for the melt consists of a quartz pipe (9) which is covered externally with a slag cap (12) and is encircled completely or partially by a metal pipe (10) which is inserted in a sealed manner into the side wall (4) of the solidification chamber (3), in which the quartz pipe is secured in the metal pipe with fireproof cement (11) and in the interior of the quartz pipe, if necessary, an oxidation means (13) is arranged.

**3.** A device according to Claims 1 and/or 2, characterised in that the two open ends of the metal casing are closed with plugs (5, 6), in which the upper plug (5) consists of a porous material, which on charging of the melt permits the outlet of air out of the sample chamber.

**4.** A device according to one or more of the preceding Claims, characterised in that the lower plug (6), facing the immersion end of the probe, on which plug the temperature measurement sensor (7) is fixed, is covered on the side facing the solidification chamber (3) with at least one layer of insulating fireproof paper (14).

**5.** A device according to one or more of the preceding Claims, characterised in that the distance between the central point of the inlet opening (9, 10) and the measurement site of the measurement sensor (7) is at least half as great as the internal diameter of the metal casing.

**6.** A device according to one or more of the preceding Claims, characterised in that the temperature measurement sensor (7) extends in a central longitudinal plane of the solidification chamber.

**7.** A device according to one or more of the preceding Claims, characterised in that the metal casing (4) surrounding the chamber consists of a pipe, preferably with a circular cross-section.

**Revendications**

**1.** Dispositif pour déterminer la teneur en carbone dans l'acier avec une teneur en carbone se situant dans une plage de 0,04 à 0,4 % par des mesures du point de liquidus, la différence maximale entre la température du métal liquide et la température de solidification de celui-ci s'élevant à 250°C, comprenant une sonde de mesure consommable avec une chambre de solidification, qui est pourvue d'une ouverture d'entrée pour le métal et d'un capteur de mesure de température, qui se projette sur l'extrémité opposée à l'ouverture d'entrée dans la chambre et avec lequel on détermine le déroulement du refroidissement du métal liquide pour une certaine durée, caractérisé par l'utilisation simultanée des caractéristiques suivantes:

a) la paroi latérale de la chambre de solidification est formée d'une enveloppe métallique (4) d'acier avec une teneur en carbone d'environ 0,04%, entourant la chambre en totalité et s'étendant dans la direction longitudinale de la sonde de mesure (1),

b) les deux extrémités ouvertes de l'enveloppe sont recouvertes d'un matériau (5, 6), dont la conductibilité thermique est substantiellement plus faible que celle du métal de l'enveloppe,

c) l'enveloppe métallique (4) est entièrement entourée sur le côté éloigné de la chambre de solidification par un matériau (2, 8), dont la conductibilité thermique est substantiellement plus faible que celle du métal de l'enveloppe (4),

d) la masse de l'enveloppe métallique (4) est mesurée de telle manière, que le rapport de la masse de l'éprouvette en acier (Mp) à la masse de l'enveloppe métallique (Mw) se situe dans une plage de 1,8 à 2,6, et la chaleur qui est apportée par la fonte s'écoulant dans la chambre de solidification (3) et est reçue par l'enveloppe métallique, mène dans la région du capteur de mesure de la température (7) à une température d'équilibre, qui est aussi proche que possible de la température de solidification.

**2.** Dispositif selon la revendication 1, caractérisé en ce que l'entrée pour la fonte consiste en un tube de quartz (9), qui est recouvert vers l'extérieur avec un capuchon de laitier (12) et est entouré entièrement ou partiellement par un tube métallique (10), qui est inséré de façon étanche dans la paroi latérale (4) de la chambre de solidification (3), et le tube de quartz est fixé dans le tube métallique avec un ciment réfractaire (11) et un produit oxydant (13) est agencé à l'intérieur du tube de quartz, si nécessaire.

**3.** Dispositif selon les revendications 1 et/ou 2, caractérisé en ce que les deux extrémités ouvertes de l'enveloppe métallique sont fermées avec des bouchons (5, 6), et le bouchon supérieur (5) est constitué d'un matériau poreux, qui permet lors du remplissage de la fonte la sortie de l'air hors de la chambre d'essai.

**4.** Dispositif selon une ou plusieurs des revendications précédentes, caractérisé en ce que le bouchon (6) inférieur, tourné vers l'extrémité plongeante de la sonde, sur lequel est monté le capteur de mesure de température (7), est recouvert sur le côté tourné vers la chambre de solidification (3) d'au moins une couche de papier isolant réfractaire (14).

**5.** Dispositif selon une ou plusieurs des revendications précédentes, caractérisé en ce que l'espacement entre le centre de l'ouverture d'entrée (9, 10) et l'emplacement de mesure du capteur de mesure (7) est au moins égal à la moitié du diamètre intérieur de l'enveloppe métallique.

**6.** Dispositif selon une ou plusieurs des revendications précédentes, caractérisé en ce que le capteur de mesure de température (7) s'étend dans un plan médian longitudinal de la chambre de solidification.

**7.** Dispositif selon une ou plusieurs des revendications précédentes, caractérisé en ce que l'enveloppe métallique (4) entourant la chambre consiste en un tube avec une section de préférence en forme de cercle.

Fig 1

EP 0 402 638 B1

Fig 2a

Fig 2b

Schnitt A-A

Fig 3

Fig 4a

Fig 4b

Fig 5a

Fig 5b

Fig 5c

EP 0 402 638 B1

Fig 5d

Fig 5e

Fig 5f